Europäisches Patentamt

European Patent Office

Office européen des brevets

(11) Publication number: **0 463 169 A1**

## EUROPEAN PATENT APPLICATION
### published in accordance with Art. 158(3) EPC

(21) Application number: 90915965.9

(22) Date of filing: **18.01.90**

(51) Int. Cl.5: **A61M 27/00**

(86) International application number:
**PCT/SU90/00015**

(87) International publication number:
**WO 91/10468 (25.07.91 91/17)**

(43) Date of publication of application:
**02.01.92 Bulletin 92/01**

(84) Designated Contracting States:
**BE CH DE FR GB IT LI SE**

(71) Applicant: **POLTAVSKY MEDITSINSKY STOMATOLOGICHESKY INSTITUT**
**ul. Shevchenko, 23,**
**Poltava 314000(SU)**

(72) Inventor: **MAZURIK, Sergei Mikhailovich**
**ul. Lenina, 92-57**
**Poltava, 314022(SU)**
Inventor: **SOKOLOV, Andrei Nikolaevich**
**ul. 60 let SSSR, 3-20**
**Poltava, 314022(SU)**

(74) Representative: **Lerwill, John et al**
**A.A. Thornton & Co. Northumberland House**
**303-306 High Holborn**
**London, WC1V 7LE(GB)**

(54) **T-SHAPED DRAINAGE TUBE.**

(57) The invention relates to medical techniques. A drainage tube comprises a longitudinal section (1) consisting of a single bore tube and a transverse section (2) shaped as a plate rolled into a tube. The plate is provided with an opening through which are interconnected sections (1) and (2) of the tube. Sections (1) and (2) are made as a single piece. The drainage tube is intended for use in hospitals dealing with bile duct surgery.

FIG.2

## Technical Field

The invention relates generally to medical engineering and more specifically to T-drain tubes made use of for drainage of the common bile duct.

## Prior Art

Known in the art is a T-drain tube made of an elastic material (latex) and applied for external drainage of extrahepatic bile ducts within the postoperative period (T-Drain, Cattell, code No 2575, product of the USA available from Inmed corporation (2950 Pacific Drive), Norcross, GA, 30071).

The drain tube in question is composed of a longitudinal member and a transverse member, which are made as an integral unit to form a T-structure. In the course of surgery the transverse member of said drain tube is situated in the common bile duct, while its longitudinal member is brought outside through a special incision made in the anterior abdominal wall. Within the postoperative period the drain tube under consideration is withdrawn from the common bile duct in such a manner that the double-lumen transverse member folded together is passed through an opening established round a single-lumen member of the T-drain tube, with the result that the walls of the common bile duct is slightly torn or lacerated and a pronounced traumatic injury to the surrounding soft tissues ensures. Moreover, it is through the gaping wound of the common bile duct a great amount of bile is free to discharge after withdrawl of the drain tube, which might be causative of diverse complications. In this respect surgeons are to place limitation upon indications for application of drainage of the common bile duct through the use of a known T-drain tube, which is in fact the optimum drainage system applied to the extrahepatic bile ducts.

## Disclosure of the Invention

It is an object of the invention to provide a T-drain tube having such a construction that would prevent injury to the walls of the common bile duct and to the surrounding soft tissues when withdrawing the T-drain tube from the common bile duct within the post-operative period.

The essence of the invention resides in that in a T-drain tube having a longitudinal portion and a transverse portion, both made as an integral unit, according to the invention, the transverse portion is shaped as a plate rolled up into a tube and having an opening through which the space confined within the dolled up plate, communicates with the lumen of the longitudinal tube portion.

It is expedient, for sake of convenience in manufacture, that the axial length of the transverse portion of a T-drain tube be equal to the circumferential length of the longitudinal tube portion.

It is practicable, for the sake of a lower degree of traumatism, that the plate corners adjacent to the longitudinal tube portion be bevelled.

The drain tube, according to the invention, prevents traumatic injury to the walls of the common bile ducts and to the surrounding tissues, when being withdrawn from the common bile duct within the postoperative period. Production of the proposed T-drain tubes is less expensive than production of those known to use presently.

## Summary of the Drawings

In what follows the invention is illustrated by a detailed description of a specific exemplary embodiment thereof with reference to the accompanying drawings, wherein:

Fig.1 is a general perspective view of a T-drain tube, according to the invention;

Fig.2 is a longitudinal sectional view of Fig.1;

Fig.3 is a view of Fig.1 with the plate unrolled; and

Fig.4 is a view of Fig.4 to illustrate the plate while in manufacture.

## Preferred Embodiment of the Invention

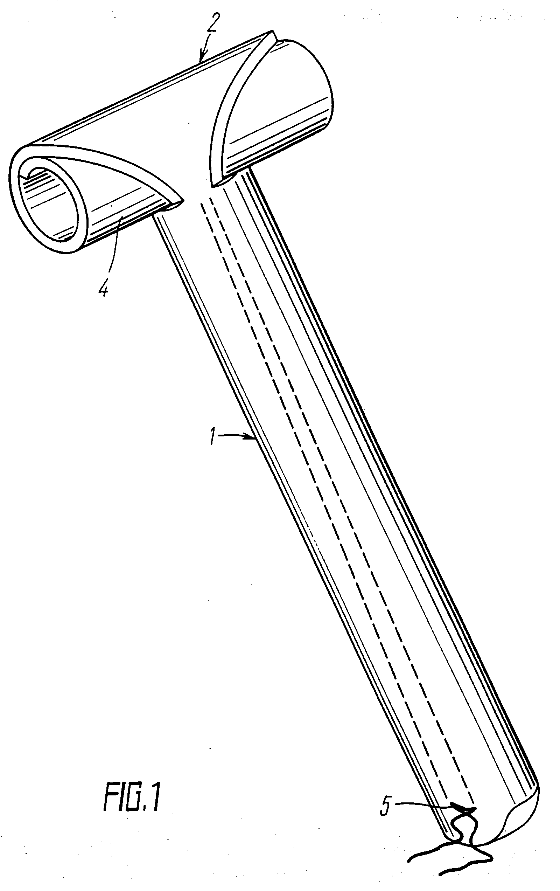

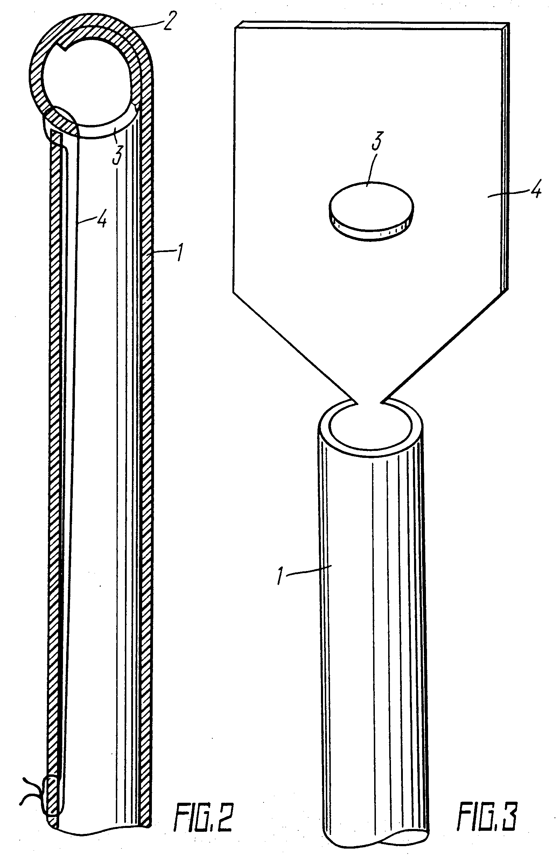

The T-drain tube as shown in Figs 1, 2 Comprises a longitudinal portion 1 and a transverse portion 2, both being made as an integral unit. The longitudinal portion 1 is essentially a tube made of an elastic material, e.g., latex, while the transverse portion 2 is made of the same material and is established by a plate rolled up into a tube having an opening 3 through which the space confined within the rolled-up plate is free to communicate with the lumen of the longitudinal portion 1.

In a specific embodiment of the drain tube described herein the axial length of the transverse portion 2 is equal to the circumferential length of the longitudinal tube portion 1, hereinafter referred to as the tube 1. However, the portion 2 may have a longer length. The corners of the plate establishing the portion 2 that are adjacent to the longitudinal portion that is the tube 1, are bevelled.

The T-drain tube, according to the invention, is produced as follows.

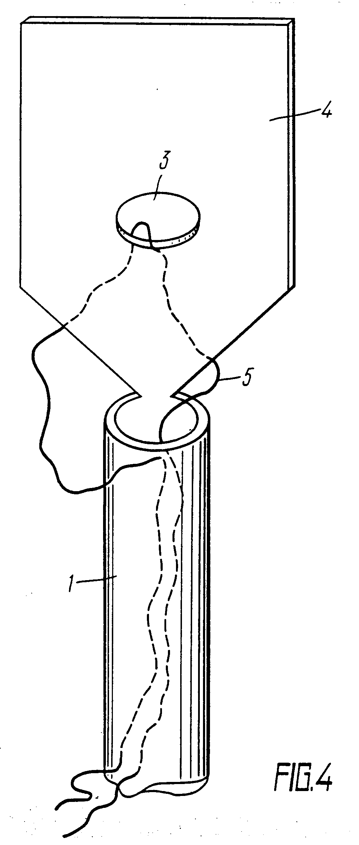

A single-lumen tube made of an elastic material, e.g., latex is cut at one of its ends along its generatrix for a length somewhat in excess of the circumferential length of the tube 1. Then the tube 1 is transected at the end point of the longitudinal cut for about one-third its circumferential length on either side of the longitudinal cut. The corners of a resultant plate 4 (Fig.4) that are adjacent to the single-lumen tube 1 are bevelled. The length of a non-bevelled edge of the plate 4 equals the circumferential length of the single-lumen tube 1. Next the opening 3 is made in the plate 4 situated along the center line thereof, whose diameter is equal to that of the single-lumen tube 1. The opening 3 is to be spaced so apart from the outer (non-bevelled) edge of the plate 4 that, when rolling up the plate 4 into a tube having a diameter equal to that of the tube 1, the opening 3 be situated opposite to the lumen of the single-lumen tube 1. Thereupon, the plate 4 is stitched with a thread 5 (Fig.4) at that edge of the opening 3 which is located nearer to the single-lumen tube 1, after which the vacant end of said tube 1 is stitched with the same thread 5 along the center line thereof, the ends of the thread 5 are passed through the lumen of the tube 1 and brought outside through the wall of the tube 1 at a distance of 10 to 12 cm by stitching. Next the plate 4 is rolled up transversely, while taking care to see that the opening 3 be located opposite to the lumen of the single-lumen tube 1, whereupon the plate 4 is fixed in that position with the thread 5 the ends of which are tied up under tension on the wall of the single-lumen tube 1 (Fig.1) The resultant structure is of the T-shape having the portions 1 and 2 made as an integral unit.

The proposed drain tube is applied as follows.

Once it has been found necessary to apply external drainage of the common bile duct the surgeon sets the atraumatic T-drain tube, according to the invention, to a fixed position, using conventional techniques. In order to withdraw the T-drain tube from the common bile duct within the postoperative period, one should to cut the thread 5 and to extract it from the tube 1 outwards. Then one should start to withdraw the drain tube by moving the single-lumen tube 1, with the result that the plate 4 is turned so first its bevelled edge passes through the fistular canal. As a result, the plate 4 is rolled up longitudinal so that the T-tube drainage system passes through the fistular canal as a conventional single-lumen tube. Thus, there is attained complete avoidance of a traumatic injury to the soft tissues when withdrawing the T-drain tube from the common bile duct.

Industrial Applicability

The drain tube is applicable in hospitals involved in surgery of bile ducts.

**Claims**

1. A T-drain tube, comprising a longitudinal portion (1) and a transverse portion (2) made as an integral unit, **characterized** in that the transverse portion (2) is shaped as a plate (4) rolled up into a tube and having an opening (3) adapted to establish communication between the space confined within said rolled-up plate (4) and the lumen of said longitudinal portion (1) of the tube.

2. A drain tube as claimed in Claim 1. **characterized** in that the transverse portion (2) has an axial length equal to the circumferential length of the longitudinal portion (1).

3. A drain tube as claimed in Claim 1, **characterized** in that the corners of the plate (4) that are adjacent to the longitudinal portion (1) are bevelled.

FIG.1

FIG.2

FIG.3

FIG.4

# INTERNATIONAL SEARCH REPORT

International Application No PCT/SU 90/00015

**I. CLASSIFICATION OF SUBJECT MATTER** (if several classification symbols apply, indicate all) *

According to International Patent Classification (IPC) or to both National Classification and IPC

Int.Cl.$^5$    A 61 M 27/00

**II. FIELDS SEARCHED**

| Minimum Documentation Searched 7 | |
|---|---|
| Classification System | Classification Symbols |
| Int.Cl.$^5$ | A 61 M 27/00 |

Documentation Searched other than Minimum Documentation
to the Extent that such Documents are Included in the Fields Searched 8

**III. DOCUMENTS CONSIDERED TO BE RELEVANT** 9

| Category * | Citation of Document, 11 with indication, where appropriate, of the relevant passages 12 | Relevant to Claim No. 13 |
|---|---|---|
| A | US, A, 4072153 (WILLIAM H. SWARTZ), 7 February 1978 (07.02.78), fig.3, column 2, lines 41,42, column 3, lines 1-5 | 1-3 |
| A | US, A, 4142528 (JOSEPH G. WHELAN et al.), 6 March 1979 (06.03.79), fig. 1, column 4, lines 38-41, column 5, lines 25-40 | 1-3 |
| A | SU, A1, 1409285 (S.M. Mazurik et al.), 15 July 1988 (15.07.88), fig. 1, column 1, lines 7-15 | 1-3 |

------------------

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 3 December 1990 (03.12.90) | 17 January 1991 (17.01.91) |
| International Searching Authority | Signature of Authorized Officer |
| ISA/SU | |

Form PCT/ISA/210 (second sheet) (January 1985)